(19) Europäisches Patentamt
European Patent Office
Office européen des brevets

(11) Numéro de publication: **0 241 376**
**A1**

(12) **DEMANDE DE BREVET EUROPEEN**

(21) Numéro de dépôt: **87400792.5**

(22) Date de dépôt: **08.04.87**

(51) Int. Cl.[4]: **A 61 K 9/50**
A 61 K 47/00, C 12 P 19/44,
C 07 H 11/04

(30) Priorité: **08.04.86 FR 8604977**

(43) Date de publication de la demande:
**14.10.87 Bulletin 87/42**

(84) Etats contractants désignés:
**AT BE CH DE ES GB GR IT LI LU NL SE**

(71) Demandeur: **CENTRE NATIONAL DE LA RECHERCHE SCIENTIFIQUE (CNRS)**
**15, Quai Anatole France**
**F-75007 Paris (FR)**

(72) Inventeur: **Barratt, Gillian, Margaret**
**20, rue d'Arcueil**
**F-75014 Paris (FR)**

**Lederer, Edgar**
**9, Boulevard Colbert**
**F-92330 Sceaux (FR)**

**Petit, Jean-François**
**24, rue Ernest Renan**
**F-75015 Paris (FR)**

**Tenu, Jean-Pierre Georges**
**3 Square des Colonnes**
**F-92360 Meudon-La-Foret (FR)**

**Yapo, Yao Alexandre**
**42, Sentier des Sablons**
**F-94230 Cachan (FR)**

(74) Mandataire: **Nony, Michel et al**
**Cabinet NONY & CIE 29, rue Cambacérès**
**F-75008 Paris (FR)**

(54) **Nouveaux liposomes à base de phosphatidylinositolmannosides, et compositions pharmaceutiques les contenant.**

(57) Utilisation, dans la préparation des liposomes, des phosphoinositolmannosides, notamment ceux extraits de mycobactéries ; liposomes contenant des phosphoinositolmmanosides, et compositions pharmaceutiques comprenant un activateur des monocytes et/ou des macrophages encapsulé dans ces liposomes.

**EP 0 241 376 A1**

**Description**

Nouveaux liposomes à base de phosphatidylinositolmannosides, et compositions pharmaceutiques les contenant.

La présente invention a pour objet de nouveaux liposomes à base de phosphatidylinositol mannosides et des compositions pharmaceutiques contenant de tels liposomes.

On sait que les phospholipides sont capables de former spontanément en milieu aqueux des vésicules constituées par des sphérules lipidiques dans lesquelles est emprisonnée une microgoutte du milieu aqueux. De telles vésicules, appelées liposomes, ont été proposées comme agents de transport des médicaments, et les recherches récentes ont principalement porté sur l'obtention de liposomes capables de ne libérer le médicament qu'ils transportent qu'au voisinage ou au contact des cellules-cibles pour ledit médicament.

La mise au point de liposomes ayant une spécificité suffisante vis-à-vis de la cellule-cible et ayant une stabilité satisfaisante in vivo soulève des problèmes techniques difficiles à résoudre.

On sait que l'activation des monocytes et/ou des macrophages constitue une des voies de recherche de la lutte contre les cellules tumorales.

On sait en effet que les macrophages activés ont un effet tumoricide. Des études réalisées in vitro ont montré que certaines substances, en particulier le muramyldipeptide (MDP) et ses dérivés, sont susceptibles d'activer les macrophages et d'induire l'action tumoricide de ceux-ci. L'utilisation comme agent activateur des monocytes et/ou macrophages du MDP ou de ses dérivés est particulièrement intéressante car il s'agit de composés qui peuvent être obtenus par synthèse. Toutefois, l'utilisation directe de ces composés in vivo n'est pas possible car ils sont excrétés très rapidement dans les urines.

On a proposé récemment d'activer les macrophages à l'aide de muramyldipeptide encapsulé dans des liposomes à base de phosphatidylcholine (PC) et de phosphatidylsérine (PS), de tels liposomes ayant été mentionnés comme particulièrement intéressants dans le cas où les cellules-cibles sont les macrophages ; voir notamment SABURO SONE et al. Recent Adv. in R.E.S. Research, 22, 187-200 (1983) et J. Immunol, 129 1313-1317 (1982) ; SCHROIT et al. Biol. Cell. 47, 87-94 (1983).

La présente invention concerne de nouveaux liposomes pouvant servir de transporteurs de drogue notamment auprès des macrophages. Une caractéristique intéressante des liposomes de l'invention est que leur capture par les macrophages n'est pas inhibée par les liposomes du type PC-PS utilisés par les auteurs qui viennent d'être cités. Autrement dit, le mécanisme de capture de ces liposomes par les macrophages est différent du mécanisme de capture des liposomes PC-PS, ce qui permet d'envisa ger l'utilisation simultanée des deux types de liposomes sans inhibition de l'un par l'autre.

Dans la recherche de liposomes susceptibles d'avoir comme cellules-cibles les macrophages, on a également proposé des liposomes constitués de phosphatidylcholine et d'un dérivé aminomannosylé du cholestérol ; voir WU et al., "Proc. Natl. Ac. Sci. U.S.A., vol. 78, n°4, PP.2033-2037 (Avril 1981)". WU et al. ont montré que ces liposomes aminomannosylés sont inhibés par les liposomes du type phosphatidylcholine/cholestérol. En outre, WU et al. ont montré que la capture des liposomes à base de phosphatidylcholine/cholestérol ou de phosphatidylcholine/cholestérol mannosylé, par les macrophages péritonéaux de souris, est extrêmement faible, contrairement à ce qui est observé avec le dérivé aminomannosylé.

On a maintenant découvert que l'utilisation de certains phospholipides mannosylés particuliers permet la réalisation de liposomes qui, de façon surprenante, ont un taux important de capture par les macrophages, non diminué par la présence de liposomes classiques du type phosphatidylcholine-phosphatidylsérine.

La présente invention a donc pour objet l'utilisation, dans la préparation de liposomes, de phosphatidylinositol mannosides.

Parmi les phosphatidylinositol mannosides, on citera notamment ceux qui sont extraits des mycobactéries. L'extraction des phosphatidylinositol mannosides des mycobactéries est connue et peut être effectuée par exemple selon les méthodes décrites par C.E. BALLOU et al., "The Journal of Biological Chemistry, Vol. 238, n°1, PP.69-76 (Janvier 1963)". Les mycobactéries de départ peuvent être par exemple Mycobacterium tuberculosis,Mycobacterium bovis (notamment bacille CALMETTE et GUERIN), Mycobacterium phlei (ATCC 354), etc.

La structure des phosphatidylinositol mannosides est connue en particulier par les études de C.E. BALLOU et al. article cité, voir aussi l'ouvrage "The Mycobacteria, a Source Book, Part A, Ed. by T. KUBICA et L. G. WAYNE, Marcel DEKKER Inc., New York et Bâle (1984), en particulier chapitre 16, PP 379-415.

L'invention a donc en particulier pour objet l'utilisation, dans la préparation de liposomes, de phosphatidylinositol mannosides de formule I

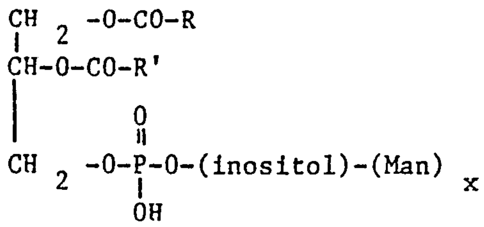

(Formule I)

dans laquelle R et R′ représentent indépendamment un groupement aliphatique ayant 11 à 25 atomes de carbone et comportant éventuellement une double liaison,
et $(Man)_x$ représente au moins un groupement mannoside lié à l'inositol, x étant le nombre de motifs de mannose.

Parmi les acides représentés par R et R′, on citera notamment l'acide myristique, l'acide palmitique, l'acide stéarique, l'acide béhénique, l'acide lignocérique, l'acide oléïque, ou un autre acide octadécènoïque, l'acide palmitoléïque, l'acide nervonique, l'acide 10-méthylstéarique.

Dans la formule I, x est de préférence un nombre variant de 1 à 5.

Parmi les phosphatidylinositol mannosides de formule I, on citera en particulier les dérivés de phosphomyoinositol de formule II

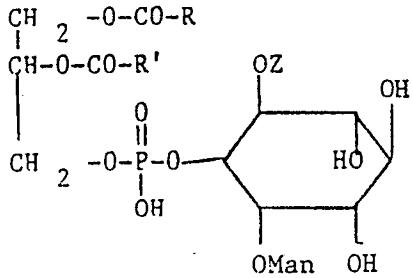

dans laquelle Z représente -H (élément hydrogène) ou $-(Man)_y$, Man étant défini comme précédemment et y étant un nombre variant de 1 à 4.

L'invention a également pour objet des liposomes préparés à l'aide des phosphatidylinositolmannosides tels que définis ci-dessus. Ils peuvent être préparés selon les méthodes classiques de préparation des liposomes ; voir par exemple L.D. Leserman et J. Barbet, Methodologie des liposomes, Séminaire technologique INSERM, les Editions de l'INSERM, PARIS.

Les phosphatidylinositol mannosides utilisés selon l'invention peuvent être également préparés par synthèse, par exemple au départ du phosphate d'inositol, qui est un produit commercial, par réaction avec un diacylglycérol, puis mannosylation, selon les méthodes connues.

Les liposomes de l'invention peuvent contenir, outre des phosphatidylinositolmannosides, d'autres phospholipides tels que la phosphatidylcholine ou la phosphatidylsérine, ainsi qu'un agent diminuant la perméabilité de la vésicule lipidique. Cet agent est notamment un stérol, par exemple le cholestérol.

Le rapport pondéral phosphatidylinositolmannoside / (agent diminuant la perméabilité) varie par exemple de 2 à 10.

L'invention a également pour objet une composition pharmaceutique caractérisée par le fait qu'elle comprend un ingrédient activateur des monocytes et/ou des macrophages encapsulé dans des liposomes.

L'ingrédient activateur est un agent capable d'augmenter l'activité anti-tumorale des macrophages.

Dans un mode de réalisation particulier, la composition pharmaceutique de l'invention est caractérisée par le fait que ledit activateur des monocytes et/ou des macrophages est le muramyldipeptide ou un de ses dérivés.

On sait que le MDP est le composé N-acétylmuramyl-L-Ala-D-isoGln.

Parmi les dérivés du MDP, on peut également citer :
un muramyltripeptide tel que le N-acétylmuramyl-L-Ala-D-isoGln-meso-$A_2$pm,un muramyltétrapeptide tel que le N-acétylmuramyl-L-Ala-D-isoGln-meso-$A_2$pm-D-Ala, un muramylpentapeptide tel que le N-acétylmuramyl-L-Ala-D-isoGln-meso-$A_2$pm-D-Ala-D-Ala.

On peut également utiliser comme dérivé du MDP, le MTPChol (MDP-L-alanyl-cholestérol) ; voir par exemple N.C. PHILLIPS et al., J. Biol. Resp. Mod. 4, 464-474 (1985).

On peut également utiliser comme médicament immunostimulant l'interféron gamma.

Les compositions immunostimulantes de l'invention sont administrées par exemple par voie intraveineuse ou intratrachéale, ou encore sous forme d'aérosols par voie intranasale.

La posologie est fonction de la drogue active utilisée. Elle est généralement égale, ou même inférieure à la posologie de ladite drogue active.

Les compositions de l'invention peuvent être utilisées notamment dans le traitement des dépressions

immunitaires, dans la lutte contre les infections (bactériennes, virales ou parasitaires) et également dans le traitement des cancers, en particulier pour prévenir ou éradiquer les métastases y compris les métastases pulmonaires.

L'invention a également pour objet l'utilisation des liposomes tels que définis précédemment notamment pour prévenir l'implantation et/ou favoriser l'éradication des métastases, en particulier des métastases pulmonaires.

Les exemples suivants illustrent l'invention sans toutefois la limiter.

EXEMPLE 1: Préparation de liposomes

On extrait les phospholipides mannosylés (en abrégé PLM) du bacille CALMETTE et GUERIN selon la méthode décrite par BALLOU et al., J. Biol. Chem., 235, pages 69-76 (1963).

On mélange les PLM avec du cholestérol (PROLABO-FRANCE) dans les proportions pondérales 2:1.

On dissout le mélange dans le chloroforme et on évapore à sec dans un flacon à fond sphérique.

On ajoute ensuite un tampon phosphate à raison d'1 ml pour 20mg de PLM.

On agite ensuite vigoureusement le flacon afin de former un vortex et détacher le film lipidique de la paroi pour former des liposomes.

Les liposomes sont ensuite centrifugés six fois à 29.000 x g pendant 25 minutes à 4° C pour éliminer la phase aqueuse non encapsulée.

Les culots de centrifugation sont à chaque fois remis en suspension dans un volume approprié de tampon phosphate.

On filtre ensuite les liposomes sur filtre d'acétate de cellulose (Millipore) de façon à éliminer ceux qui ont un diamètre supérieur à 3µm.

Obtention de liposomes marqués

Pour pouvoir procéder à diverses déterminations, en particulier pour mesurer le taux d'encapsulation, on a préparé de façon analogue des liposomes marqués avec un agent radioactif dans la phase lipidique et/ou dans la phase aqueuse.

Pour marquer la phase lipidique on a ajouté aux PLM des traces de $^{14}$C-DPPC (dipalmitoyl-1-($^{14}$C) phosphatidylcholine ; New England Nuclear ; 0,01 mCi/mmol) dans la solution de chloroforme.

Pour marquer la phase aqueuse, on a ajouté des traces de ($^{3}$H)-sucrose (Amersham International ; 9,8 Ci/mmol).

Taux d'encapsulation

On mélange 50mg de PLM avec 25mg de cholestérol et 0,5µCi de $^{14}$C-DPPC dans 10ml de chloroforme.

Comme précédemment, on forme un film par évaporation puis on ajoute 5ml de tampon PBS contenant 150µCi de sucrose tritié. Après plusieurs centrifugations comme précédemment, le culot est remis en suspension dans 1ml de PBS puis filtré sur membrane Millipore.

Le taux d'encapsulation est exprimé en microlitres de phase aqueuse par mg de phospholipides.

Après l'étape de centrifugation, on a trouvé un taux d'encapsulation de 7,9.

Après l'étape de filtration sur filtres Millipore, on a trouvé un taux d'encapsulation de 9,5.

Stabilité des liposomes PLM : cholestérol

Dans une expérience préliminaire, on a préparé de façon analogue à celle décrite précédemment des liposomes PLM sans cholestérol et avec cholestérol (proportions pondérales PLM-cholestérol 2:1).

La fuite de la phase aqueuse est mesurée par la fuite de radioactivité (sucrose tritié) après incubation pendant 30 min à 37° C en milieu contenant 5% de sérum de veau foetal. Le pourcentage de fuite pour les liposomes PLM représente 67% de la phase aqueuse totale tandis qu'avec les liposomes PLM:cholestérol, la fuite n'est que de 6%.

EXEMPLE 2: Mesure de l'incorporation des liposomes par les macrophages

a) Obtention des macrophages inflammatoires de souris

On provoque chez des souris un exsudat inflammatoire par injection de 1,5ml d'un milieu contenant du thioglycolate. Quatre jours plus tard, on collecte les cellules de l'exsudat péritonéal. La cavité péritonéale est lavée avec du milieu MEM (Institut PASTEUR - France) et on réunit l'exsudat et les liquides de lavage. On sépare et on recueille les cellules par centrifugation à 200 x g pendant 10 min à 4° C. On remet les cellules en suspension dans le milieu MEM contenant des antibiotiques et du sérum de veau foetal à complément inactivé. On ajuste la suspension à 2.10$^{6}$ macrophages/ml. Le nombre de macrophages est évalué par incorporation de rouge neutre. On dépose 2ml de la suspension dans des boîtes de Petri de 35mm de diamètre et on laisse incuber à 37° C pendant 3 heures sous atmosphère humide à 5% de $CO_2$ pour permettre l'adhérence des macrophages. On élimine les cellules non adhérentes par trois lavages avec un tampon phosphate.

b)Préparation de macrophages alvéolaires de rats

Sur des rats anesthésiés et saignés à l'aorte abdominale, on prélève les poumons et on effectue des lavages via la trachée avec 6 x 5ml d'une solution aqueuse de chlorure de sodium à 0,9% (poids/vol.). On

centrifuge à 200 x g à 4° C pendant 15 mn. On réunit les liquides de lavage et on soumet à une centrifugation en présence de 5ml de milieu MEM contenant 5% de sérum de veau foetal et des antibiotiques. Le culot de centrifugation est remis en suspension dans le même milieu MEM et on ajuste la concentration à $10^6$ macrophages par ml. Comme précédemment, on dépose 2ml de la suspension dans des boîtes de Petri en laissant incuber pour permettre l'adhérence des cellules puis on effectue des lavages pour éliminer les cellules non adhérentes.

c) Mesure de la capture des liposomes par les macrophages

On met en suspension les liposomes dans le milieu MEM contenant 5% de sérum de veau foetal et des antibiotiques.

On ajuste la concentration en liposomes à une valeur comprise entre 50µg/ml et 400µg/ml en phospholipides.

On ajoute 2ml du milieu contenant les liposomes aux préparations de macrophages obtenues précédemment et on laisse incuber à 37° C sous une atmosphère humide contenant 5% de $CO_2$. Après la période d'incubation, on élimine le milieu liquide et on lave les macrophages quatre fois avec un tampon phosphate.

On ajoute ensuite 0,6ml d'un tampon phosphate contenant 1% (v/v) de Triton-X-100 et on recueille les macrophages avec une raclette de caoutchouc. On répète l'opération avec à nouveau 0,6ml de tampon contenant du Triton-X-100. On réunit les deux lysats obtenus.

Résultats

a) pour les macrophages inflammatoires de souris : influence du temps d'incubation.

On a fait incuber les macrophages ($4.10^6$/boîte) avec 2ml de milieu à 50µg/ml de liposomes PLM:cholestérol 2:1 en poids, les liposomes ayant un diamètre moyen de 500nm.

Les résultats sont résumés dans le tableau I suivant :

TABLEAU I

| Temps d'incubation (heures) | Quantité de lipides incorporés (microgrammes) |
|---|---|
| 2 | 2,0 |
| 4 | 3,6 |
| 6 | 6,1 |
| 20 | 18,8 |

b) Incorporation des liposomes par les macrophages inflammatoires de souris: Influence de la concentration

On a préparé des liposomes PC/PS (phosphatidylcholine : phosphatidylsérine) de façon classique dans les proportions molaires 7:3 ; cette proportion est recommandée par SCHROIT et al., article cité.

On a comparé ces liposomes avec des liposomes PLM:cholestérol 2:1.

On a incubé les macrophages avec 2ml de la suspension de liposomes dans le milieu de culture.

Chaque préparation de liposomes a été marquée avec de la $^{14}C$ -dipalmitoylphosphatidylcholine.

Les liposomes, après filtration, avaient un diamètre moyen de 500nm.

Les macrophages, à raison de $4.10^6$ cellules/boîtes de Petri, ont été incubés pendant 2 heures avec les liposomes à diverses concentrations.

Les résultats son résumés dans le tableau II suivant :

TABLEAU II

| Concentration des liposomes (µg de phospholipides/ml) | Quantité de phospholipides incorporée (µg) Liposomes PLM | Liposomes PC/PS |
|---|---|---|
| 50 | 3,7 | 1,8 |
| 100 | 6,7 | 2,7 |
| 200 | 8,9 | 3,9 |
| 400 | 18,5 | 6,8 |

c) Incorporation des liposomes par les macrophages alvéolaires de rats

L'incorporation des liposomes qui ont un diamètre moyen de 500 nm et qui sont marqués à la $^{14}C$ dipalmitoyl-phosphatidylcholine a été étudiée avec des liposomes PLM:cholestérol 2:1 et comparée à celle des liposomes PC:PS.

Les résultats sont résumés dans le tableau III :

TABLEAU III

| Microgrammes/ ml de phospholipides | Microgrammes de lipides incorporés par $2.10^6$ macrophages après 16 Heures Liposomes PLM:Cholestérol | Liposomes PC:PS |
|---|---|---|
| 50 | 3,9 | 1,6 |
| 400 | 15,3 | 8,1 |

d) Essais d'inhibition

L'addition d'un excès de liposomes PLM:cholestérol non marqués pendant l'endocytose des mêmes liposomes marqués inhibe l'incorporation de ces derniers par les macrophages.

Par exemple, on utilise des macrophages inflammatoires de souris, à raison de $4 \times 10^6$ cellules par boîte de Petri, en utilisant des liposomes PLM:cholestérol 2:1, marqués au carbone 14 comme précédemment, ainsi que les mêmes liposomes non marqués et des liposomes PC:PS 7:3 (proportions molaires). Tous les liposomes ont été filtrés et avaient un diamètre moyen de 500nm. Les liposomes marqués sont ajoutés aux macrophages dans le milieu de culture à raison de 50µg de phospholipides/ml, soit seuls (essai témoin), soit avec l'une ou l'autre des préparations non marquées présente à raison 400µg de phospholipides/ml.

Après 2 heures, les cellules sont lavées et la quantité de lipides marqués incorporés est déterminée.

Le pourcentage d'inhibition est défini de la façon suivante :

$$\% \text{ d'inhibition} = (1-\frac{A}{B}) \times 100$$

avec A = quantité incorporée en présence de liposomes non marqués
et B = quantité incorporée dans l'essai témoin.

Les résultats sont résumés dans le tableau IV suivant :

TABLEAU IV

| Nature des liposomes non marqués | µg de phospholipides marqués incorporés | % d'inhibition |
|---|---|---|
| aucun (témoin) | 3,8 | - |
| PLM:cholestérol | 1,0 | 74 |
| PC:PS | 3,8 | 0 |

On voit que les liposomes PC:PS n'ont aucun effet inhibiteur sur l'incorporation des liposomes PLM:cholestérol. Il apparaît donc que les deux types de liposomes sont incorporés selon des mécanismes différents.

EXEMPLE 3

Activation des macrophages alvéolaires de rats in vitro

Dans cet essai, on a utilisé un dérivé lipophile du MDP : il s'agit du dérivé MDP-L-alanyl-cholestérol (en abrégé MTPChol), dont l'activité a été démontrée dans d'autres systèmes ; voir N.C. Phillips et al., J. Biol. Resp. Mod. 4, 464-474 (1985).

On a préparé des liposomes contenant les phosphatidylinositolmannosides (PLM) et du cholestérol dans les proportions pondérales 2:1 et on a incorporé le MTPChol à une concentration de 1µg pour 120µg de phospholipides. Pour comparaison, on a préparé des liposomes analogues sans MTPChol.

Les liposomes ont été préparés dans des conditions stériles et filtrés de façon à obtenir un diamètre moyen de 440nm.

Les macrophages alvéolaires de rats sont mis dans des cupules d'une boîte à 96 puits à raison de 0,25 ml par puits d'une suspension contenant 8 x $10^5$, 4 x $10^5$ ou 2 x $10^5$ macrophages par ml. Après l'adhérence, on ajoute 0,25 ml du milieu : soit le MEM seul, soit le MEM contenant des liposomes PLM : cholestérol "vides"(sans MTPChol), ou contenant des liposomes PLM : cholestérol avec MTPChol. On incube à 37°C pendant une nuit, ensuite on lave et on ajoute les cellules cibles syngéniques (Fibrohistiocytome P77) à raison de $10^5$ par ml (afin de donner des rapports Effecteurs / Cibles de 8,4 et 2) en même temps qu'une solution de thymidine marquée avec le tritium ($^3$H-TdR) à une concentration finale de 1.2 µM de thymidine. On incube encore 20 h à 37°C, puis on récolte l'ADN des cellules tumorales et on dose l'incorporation de précurseur marqué à l'aide d'un spectromètre β à scintillation liquide.

Résultats

Une activation des macrophages se traduit par une inhibition de croissance des cellules tumorales (diminution d'incorporation de $^3$H-TdR) cultivées en présence des macrophages par rapport aux cellules tumorales cultivées seules. Les macrophages traités avec le milieu seul ne sont pas activés. De même, les macrophages préincubés avec les liposomes vides, de 20µg/ml à 400µg/ml de phospholipides, ne provoquent pas une inhibition significative de la croissance de la cible. En revanche, à toutes les concentrations citées, les liposomes contenant le MTPChol activent les macrophages, qui sont ensuite capables d'inhiber la croissance des cellules tumorales d'une façon importante. Le tableau V montre les resultats avec 20µg de phospholipides/ml de liposomes contenant ou non le MTPChol (0,17 µg/ml).

TABLEAU V

| Prétraitement des Macrophages | Croissance des cellules cibles (% tumeur seule) avec des macrophages à un rapport Effecteur/Cible de 2 | 4 | 8 |
|---|---|---|---|
| Liposomes "vides" | 74 | 68 | 72 |
| Liposomes-MTPChol | 47 | 13 | 10 |

EXEMPLE 4

Activation de macrophages alvéolaires in vivo

On a utilisé des liposomes préparés comme dans l'exemple 3, avec et sans MTPChol. On a injecté à des rats, par voie intraveineuse, 10 mg/kg de PLM sous forme de liposomes PLM : cholestérol, contenant ou non 0,08 mg/kg MTPChol.

24 heures plus tard, on a sacrifié ces rats et récolté les macrophages alvéolaires qui ont été testés pour leur pouvoir cytostatique contre les cellules cibles syngéniques, comme cela a été décrit ci-dessus.

Résultats

Les résultats sont exprimés en pourcentage d'inhibition de croissance (% I.C.) où :

% I.C. = (1-X/R) x 100

X étant l'incorporation de $^{3}$H-TdR par les cellules cibles cultivées en présence des macrophages récoltés des rats traités par les liposomes. R étant l'incorporation de $^{3}$H-TdR par les cellules cibles cultivées en présence des macrophages des rats témoins.

On note que, bien que les liposomes "vides" soient capables d'activer un peu les macrophages, l'incorporation du MTPChol dans les liposomes produit une activation importante (Tableau VI).

TABLEAU VI.

| Traitement des rats | % I.C. en présence des macrophages à un rapport Effecteur/Cible 4 | 8 |
|---|---|---|
| Liposomes "Vides" | 3 | 19 |
| Liposomes-MTPChol | 43 | 48 |

EXEMPLE 5

Effets d'un traitement avec les liposomes contenant un dérivé de MDP sur la formation de métastases pulmonaires in vivo

On a injecté à des rats, par voie intraveineuse, des cellules tumorales syngéniques (cellules de fibrohistiocytome P77) à raison de 5.10$^{5}$ cellules par rat. On a traité un groupe de rats avec les liposomes contenant le MTPChol à une dose de 6mg/kg de phospholipides, soit 0,05mg/kg de MTPChol, injectés par

voie intraveineuse dans 0,35ml de tampon PBS. Les injections ont été faites aux jours -1, 3, 7, 10 et 14, la tumeur étant injectée au jour 0.

Un second groupe de rats a reçu des liposomes "vides" (sans MTPChol) ; enfin un troisième groupe (groupe témoin) n'a reçu aucun traitement. Dix-huit jours après l'implantation de la tumeur, on a sacrifié les rats de chaque groupe et on a compté le nombre de métastases pulmonaires visibles.

Les résultats sont résumés dans le tableau VII suivant :

TABLEAU VII

| Traitement | nombre de métastases (moyenne ± écart type) |
|---|---|
| Aucun | 96 ± 18 |
| Liposomes "vides" | 90 ± 5 |
| Liposomes-MTPChol* | 48 ± 12 |

* $p < 0{,}002$ d'après le test t de Student

Ces résultats montrent que les liposomes "vides" n'ont aucun effet sur la dissémination de la tumeur tandis que les liposomes contenant le MTPChol ont réduit de 50% le nombre des métastases visibles.

**Revendications**

1. Utilisation, dans la préparation de liposomes, de phosphatidylinositolmannosides.

2. Utilisation selon la revendication 1, caractérisée par le fait que lesdits phosphatidylinositolmannosides sont extraits de mycobactéries.

3. Utilisation selon la revendication 1 ou 2, caractérisée par le fait que lesdits phosphatidylinositolmannosides répondent à la formule

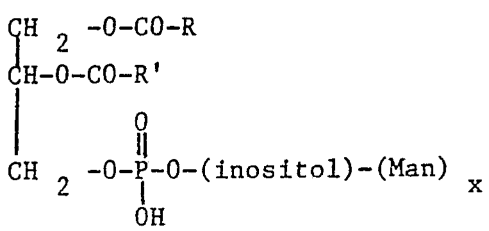

dans laquelle R et R′ représentent indépendamment un groupement aliphatique ayant 11 à 25 atomes de carbone et comportant éventuellement une double liaison,
et $(Man)_x$ représente au moins un groupement mannoside lié à l'inositol x étant le nombre de motifs de mannose.

4. Utilisation selon la revendication 3, caractérisée par le fait que lesdits groupements R et R′ représentent indépendamment les restes d'un acide choisi parmi l'acide myristique, l'acide palmitique, l'acide stéarique, l'acide béhénique, l'acide lignocérique, un acide octadécènoïque tel que l'acide oléïque, l'acide palmitoléïque, l'acide nervonique et l'acide 10-méthylstéarique.

5. Utilisation selon la revendication 3 ou 4, caractérisée par le fait que x est un nombre variant de 1 à 5.

6. Utilisation selon l'une quelconque des revendications précédentes, caractérisée par le fait que ledit phosphatidylmyoinositolmannoside répond à la formule :

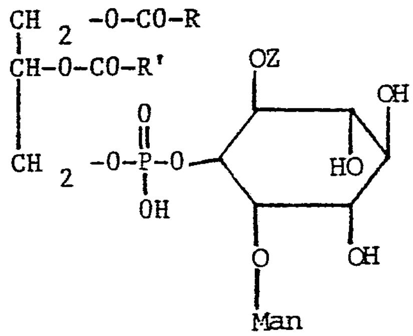

dans laquelle R et R′ sont définis comme dans la revendication 3 ou 4, Z représente -H ou - $(Man)_y$,Man est défini comme dans la revendication 3 et y est un nombre variant de 1 à 4.

7. Liposomes à base de phospholipides, caractérisés par le fait que lesdits phospholipides sont tels que définis dans l'une quelconque des revendications 1 à 6.

8. Liposomes selon la revendication 7, caractérisés par le fait qu'ils comportent en outre un agent diminuant la perméabilité de la vésicule lipidique.

9. Liposomes selon la revendication 8, caractérisés par le fait que ledit agent diminuant la perméabilité est un stérol, en particulier le cholestérol.

10. Liposomes selon la revendication 8 ou 9, caractérisés par le fait que le rapport pondéral phospholipides/agent diminuant la perméabilité varie de 2 à 10.

11. Composition pharmaceutique immunostimulante caractérisée par le fait qu'elle comprend un ingrédient activateur des monocytes et/ou des macrophages encapsulé dans des liposomes tels que définis dans l'une quelconque des revendications 7 à 10.

12. Composition selon la revendication 11, caractérisée par le fait que ledit activateur des monocytes et/ou macrophages est le muramyldipeptide ou un de ses dérivés, ou l'interféron gamma.

Office européen des brevets

# RAPPORT DE RECHERCHE EUROPEENNE

Numero de la demande

EP 87 40 0792

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 105, no. 26, 19 décembre 1986, page 360, résumé no. 232396y, Columbus, Ohio, US; G. BARRATT et al.: "Preparation and characterization of liposomes containing mannosylated phospholipids capable of targeting drugs to macrophages", & BIOCHIM. BIOPHYS. ACTA 1986, 862(1), 153-64<br>* Résumé *<br>--- | 1-12 | A 61 K 9/50<br>A 61 K 47/00<br>C 12 P 19/44<br>C 07 H 11/04 |
| Y | CHEMICAL ABSTRACTS, vol. 103, no. 11, 16 septembre 1985, page 244, résumé no. 83625b, Columbus, Ohio, US; N.M. WASSEF et al.: "Specific binding of concanavalin A to free inositol and liposomes containing phosphatidylinositol", & BIOCHEM. BIOPHYS. RES. COMMUN. 1985, 130(1), 76-83<br>* Résumé *<br>--- | 1-12 | DOMAINES TECHNIQUES RECHERCHES (Int. Cl.4)<br>A 61 K<br>C 12 P<br>C 07 H |
| Y | EXPERIENTIA, vol. 31, fasc. 7, partie II, 15 juillet 1975, pages 776-778, Birkhäuser Verlag, Basel, CH; K.R. DHARIWAL et al.: "Phospholipids of Mycobacterium phlei"<br>* En entier *<br>--- -/- | 1,2,11,12 | |

Le présent rapport de recherche a été établi pour toutes les revendications

| Lieu de la recherche | Date d'achèvement de la recherche | Examinateur |
|---|---|---|
| LA HAYE | 08-07-1987 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

**RAPPORT DE RECHERCHE EUROPEENNE**

Numéro de la demande

EP 87 40 0792

**DOCUMENTS CONSIDERES COMME PERTINENTS**



| Catégorie | Citation du document avec indication, en cas de besoin, des parties pertinentes | Revendication concernée | CLASSEMENT DE LA DEMANDE (Int. Cl.4) |
|---|---|---|---|
| Y | DE-A-2 546 001 (INSTITUT PASTEUR)<br>* Page 1, alinéa 1; page 4; revendications 1,13 *<br>--- | 1,3-7, 11,12 | |
| X | CHEMICAL ABSTRACTS, vol. 101, no. 21, 19 novembre 1984, page 399, résumé no. 187605v, Columbus, Ohio, US; J. DE BONY et al.: "Evidence for a homogeneous lateral distribution of lipids in a bacterial membrane. A photocrosslinking approach using anthracene as a photoactivable group", & FEBS LETT. 1984, 174(1), 1-6<br>* Résumé *<br>--- | 1,3-7 | |
| A | CHEMICAL ABSTRACTS, vol. 101, no. 21, 19 novembre 1984, page 536, résumé no. 189141q, Columbus, Ohio, US; L. KHANDKE et al.: "Phosphatidylinositomannosides in lepromatous leprosy nodules", & INDIAN J. MED. RES. 1984, 80(Sept.) 259-63<br>* Résumé *<br>--- -/- | 1,2 | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 08-07-1987 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82

Office européen des brevets

# RAPPORT DE RECHERCHE EUROPEENNE

Numéro de la demande

EP 87 40 0792

## DOCUMENTS CONSIDERES COMME PERTINENTS



| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 95, no. 7, 17 août 1981, page 232, résumé no. 56774j, Columbus, Ohio, US; F. LAKHDAR-GHAZAL et al.: "Phase behavior in monolayers and in water dispersions of mixtures of dimannosyl diacylglycerol with phosphatidylglycerol. Effect of monovalent and bivalent cations", & BIOCHIM. BIOPHYS. ACTA 1981, 644(2), 284-94<br>* Résumé *<br>----- | 1,3-7 | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 08-07-1987 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82